# EUROPEAN PATENT SPECIFICATION

(11) **EP 2 345 330 B1**
(45) Date of publication and mention of the grant of the patent: **19.08.2015**
(21) Application number: 11150445.2
(22) Date of filing: 10.01.2011
(51) Int. Cl.: A21C 9/00, B07C 5/342, G01N 21/89

(54) **A quality production surveillance system of elongate pasta food products**
System zur Produktionsqualitätsüberwachung von länglichen Nudelnahrungsmitteln
Système de surveillance de la production de qualité de produits alimentaires de pâte allongés

(30) Priority: 19.01.2010 IT RE20100003
(43) Date of publication of application: 20.07.2011
(73) Proprietor: R A M Elettronica S.r.l., 70031 Andria (BA) (IT); Ricciarelli S.p.a., 51100 Pistoia (IT)
(72) Inventor: Pani, Paolo, 51100, Pistoia (IT); Scarcelli, Riccardo, 70031, Andria (Bari) (IT)
(74) Representative: Corradini, Corrado

(56) References cited:
- EP-A2- 1 521 055
- DE-U1- 29 920 106
- JP-A- 2003 035 675
- JP-A- 2005 324 101
- US-A- 2 726 762
- US-A- 5 456 931
- US-A- 5 462 176
- US-A1- 2006 016 735

## Description

The invention generally relates to production plants for comestible pasta in elongate form, i.e. spaghetti, linguine, bucatini. The invention especially relates to elongate and thin products able to roll on a flat surface.

In production lines, pasta, once drawn, is "hung" on rods which travel, drawn by lateral chains, internally of a drying tunnel in which the various stages of the process are performed.

Once the drying process has been completed and the pasta is ready for packing, it is removed from the rods and unloaded onto a conveyor belt. From the belt, after having been cut to the standard length (about 260 mm for spaghetti), the pasta is sent on to the packing line, using a device known as a loader.

During the course of the process there is a risk that the product might be contaminated by residues or impurities in general, or that it might exhibit visible drying defects (cracking, white spotting, etc.).

These defects, given the high production potential of present production lines (over 4500 Kg per hour) are very difficult to observe by a human being, and unfortunately if contaminated or defective products reach the final consumer the consequences are very serious.

Pasta producers are therefore forced to reject whole production batches when there is the suspicion that these defects exist or even their sporadic presence, , since the most stringent distributor will not usually accept placing defective products up for sale.

In the patent publication JP-2005 324101-A, a quality production surveillance system of elongate shape comestible pasta is disclosed, which comprises: means for causing indeterminate quantities of product to run in a form of a substantially flat and continuous layer within an inspection space; illuminating means for illuminating the product running in the inspection space, and two television cameras able to acquire a continuous succession of images of the running product. A product expulsion group acting on a normal flow of the product is placed downstream inspection space, to deviate, on command, a limited quantity of the product towards a discharge.

An electronic processing device to control images acquired by the television cameras, commands the activation of the product expulsion group when it detects at least a single defective product, such as to expel a limited mass of product containing the single defective product.

However, the system is not able to control the whole surface of the items of the product, as these may have defects on a part of the surface hidden with respect to the cameras.

An aim of the present invention is to realise an improved system able to cause the camera to see as much as possible any defective products in the whole surface, operating automatically on the production line, which is preferably located just upstream of the packing stage.

This and other aims are attained by the present invention, as it is characterised in the accompanying claims.

The invention is described in detail in the following with the aid of the accompanying figures of the drawings, which illustrate an embodiment thereof by way of non-limiting example.
Figure 1 is a schematic section, according to an axial vertical plane, of the system of the invention.
Figure 1A is an enlarged detail of figure 1.
Figure 2 is a functioning diagram of the system of figure 1.
Figure 3 is a plan view from above of figure 1.

The surveillance system of the invention is preferably arranged as near as possible to the packing point, for example upstream of the packing machine. The system of the invention comprises means for causing undetermined quantities of product 9 to run in the form of a substantially flat and continuous layer within an inspection space 20.

In particular, the system comprises an inspection surface 11 and the moving means for causing the product 9 to run are destined to advance the product 9, arranged with the axis thereof horizontal and transversal to the advancement direction, and to release the product 9 onto the inspection surface 11 such as to realise on the said surface 11 substantially a single layer that runs substantially continuously on the surface 11 itself.

In detail, in the embodiment illustrated in the figures, the moving means for realising the running comprise a slab 12 inclined downwards in the advancement direction, the upper surface of which defines the inspection surface 11, and a conveyor belt 13 device, located upstream of the inclined slab 12, destined to transfer the product 9 and to release the product 9 onto the slab 12, in the form of a substantially single and continuous layer. The conveyor belt 13 brings the thus-distributed product 9 resting on the belt up to the final end, then releases the product 9 so causing it to fall and run on the surface 11 in a substantially continuous layered arrangement in a substantially single layer. To do this, the conveyor belt 13 is preferably continuously speed-adjustable; further, there can be provided means (not illustrated in the figures) acting on the product 9 located on the conveyor belt 13 which means can spread and distribute the product 9 uniformly and in a single layer on the belt 13.

An inclined chute (not illustrated in the figures) can be interposed between the conveyor belt 13 and the inspection surface 11, which chute can supply the surface 11 with items of the product 9 which are rolling with a rotation about its own axis as well as translating.

As an alternative embodiment, the same upper surface 11 (i.e. the inspection surface) causes the items of the product to roll with a rotation about its own axis, as well as translating, because of friction contact between the same surface and the surface of the items.

The system of the invention further comprises a transfer device 30 of the product 9, located downstream of the inspection space 20 and the inspection surface 11, destined to receive the inspected product 9, and an expulsion group of the inspected product, acting on the normal flow of the product 9 along the transfer device 30, in order on command to deviate a limited quantity thereof containing the defective product 9 towards a discharge.

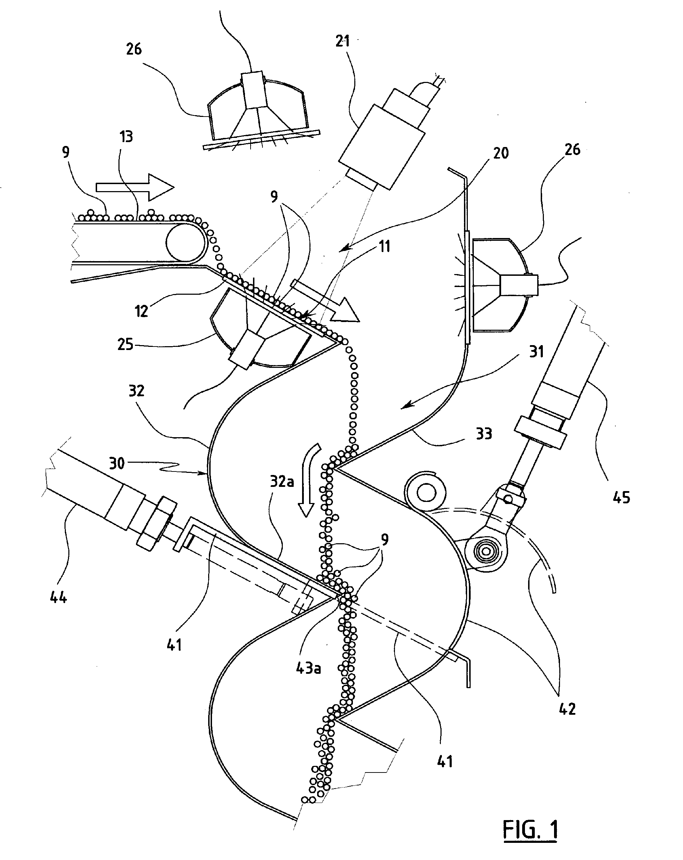

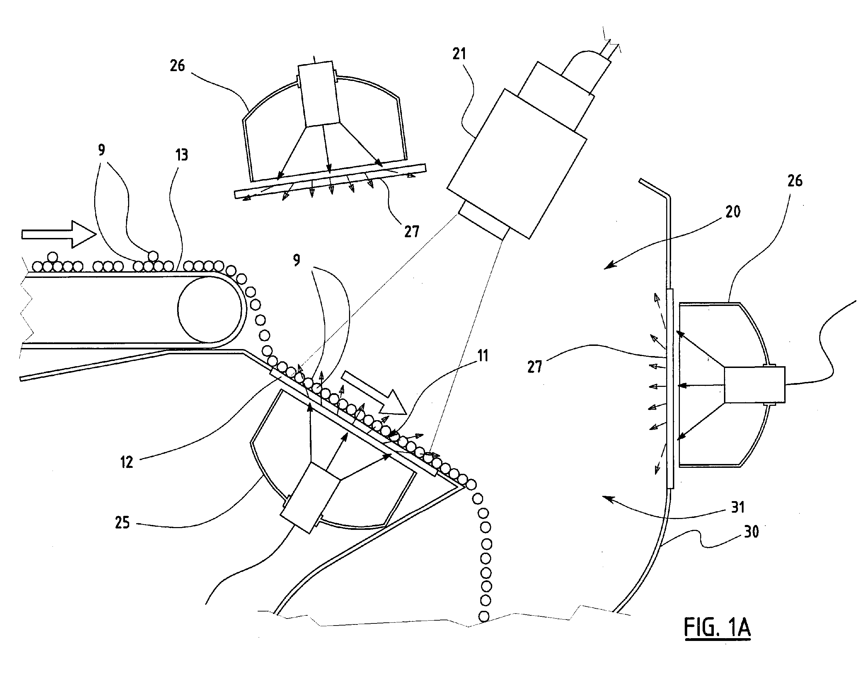

In particular, the transfer device 30 comprises a descent corridor 31, having a closed section, orientated globally such as to transfer the product 9 in a downwards direction; in particular, the longitudinal axis of the descent corridor 31 has a zigzag profile which descends, and the front wall 32 and rear wall 33 exhibit a zigzag profile in the vertical section parallel to the advancement direction of the conveyor belt 13 (as illustrated in figure 1). The device 30 is constituted by a chute device of known type, used for realising the descent of long pasta products. The product 9, coming from the inspection surface 11, descends along the corridor 31, falling, in brief tracts, from the front wall 32 to the rear wall 33 and vice versa.

The product expulsion group comprises an obturator 41 insertable and de-insertable across the descent corridor 31, destined to intercept the flow of the product 9 along the corridor 31; the expulsion group further comprises a hatch 42 afforded in a wall 32, 33 of the corridor, at the obturator 41 position, destined to evacuate the product intercepted by the obturator 41 towards the outside.

In detail, the obturator 41 comprises an inclined plate 43 destined to occupy the whole section of the corridor 31, activated by a linear actuator 44, with a translation movement that is parallel to the inclined axis of the plate 43. The plate 43 is normally arranged completely externally of the corridor 31, in order not to obstruct the flow of the product 9; when the linear actuator 44 thereof is activated, it enters the corridor 31 through an opening 43a afforded in the front wall 32 (or the rear wall 33); the insertion movement of the plate across the corridor 31 has a downwards-inclined direction; the insertion section is located at a point in which the product 9, after having rolled and moved along a tract 32a (see figure 1) of the front wall 32 (or the rear wall 33) having an internal surface facing upwards, leaves the tract 32a, which changes direction sharply, and falls on the opposite wall 33; the opening 43a is located beneath the point at which the tract 32a changes direction.

The hatch 42 is located on the opposite wall 33 to the wall affording the opening 43a, and a lower end thereof meets the point of the wall 33 against which the free end of the plate 43 rests, when the obturator is inserted; the hatch 42 is activated with a rotating motion by a linear actuator 45.

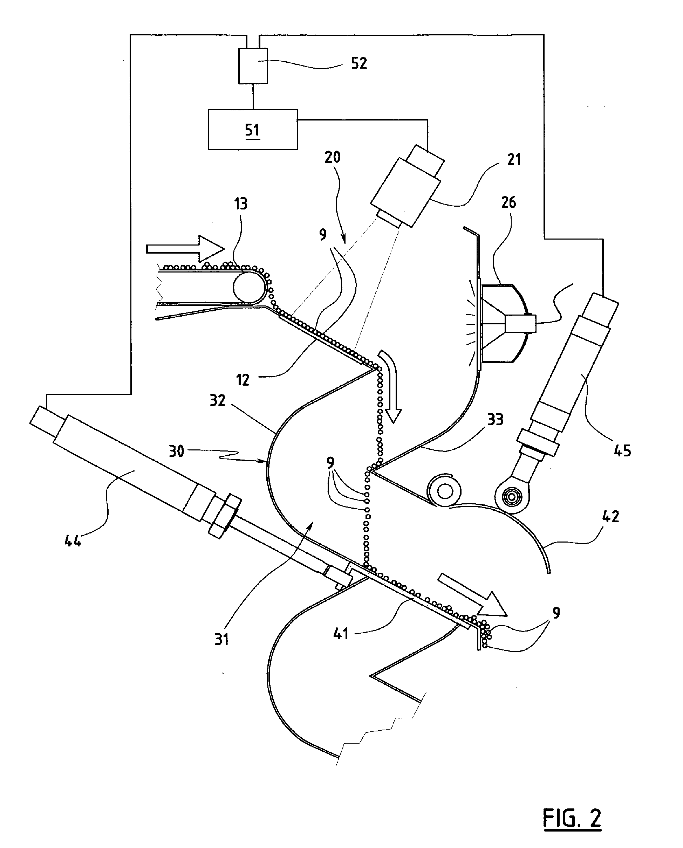

In order to evacuate a quantity of product 9 from the corridor 31, the obturator 41 is activated such as to intercept the flow of product 9, and more or less at the same time the hatch 42 is raised such as to deviate, externally of the corridor 31, the part of product 9 intercepted (see figure 2).

The invention comprises means destined to illuminate the product 9 running on the inspection surface 11 in the inspection space 20, and at least a television camera 21, destined to acquire a succession of images of the product 9 running on the inspection surface 11.

The illuminating means are designed to realise as diffused and uniform an illumination as possible, in order to prevent shadows as much as possible. The slab 12 is translucent and at least an illuminating device 25 is directed against it, which illuminating device 25 is directed from the lower surface towards the upper surface 11 of the slab 12. Further, at least an illuminating device 26, and preferably two, are provided, destined to provide a substantially diffuse light in the inspection space 20 located above the inspection surface 11, each of which preferably two illuminating devices 25 exhibits a translucent screen 27 for diffusing the light located between the light source and the inspection space 20.

The TV camera 21 is preferably of an electronic CCD type, able to acquire images in a continuous sequence, with intervals in the range of milliseconds. In order to obtain a good result in the surveillance of the product, it is important that the moving means should be able to cause the products to roll one by one, with a rotation about an axis thereof, on the surface 11, such that a whole surface thereof is exposed, along its rolling, to the TV camera, i.e. without there remaining any hidden zones of the product 9.

Also provided is an electronic processing device 50 destined to control the images acquired by the TV camera 21 and to command activation of the product expulsion group, when it detects at least a single defective pasta item in the product; the activation is commanded in such a way as to expel a limited mass of product containing the defective item.

In more detail, the pasta rolls on the surface 11 in the TV camera field; the processor 51 compares the images acquired of the product rolling on the surface 11 with reference images; when, on the basis of this comparison, the presence of zones of anomalous colour is detected, which represent a defect in the product 9, an intervention device 52 activates an intervention of the expulsion group 41, 42, such that the group 41, 42 causes expulsion of a possibly small quantity of product, such as to comprise, with a good degree of accuracy, the defective item that was detected.

Once the presence of a defective pasta item has been detected, the discharge device enables elimination of only and exclusively a relatively small number of unsuitable items to be eliminated.

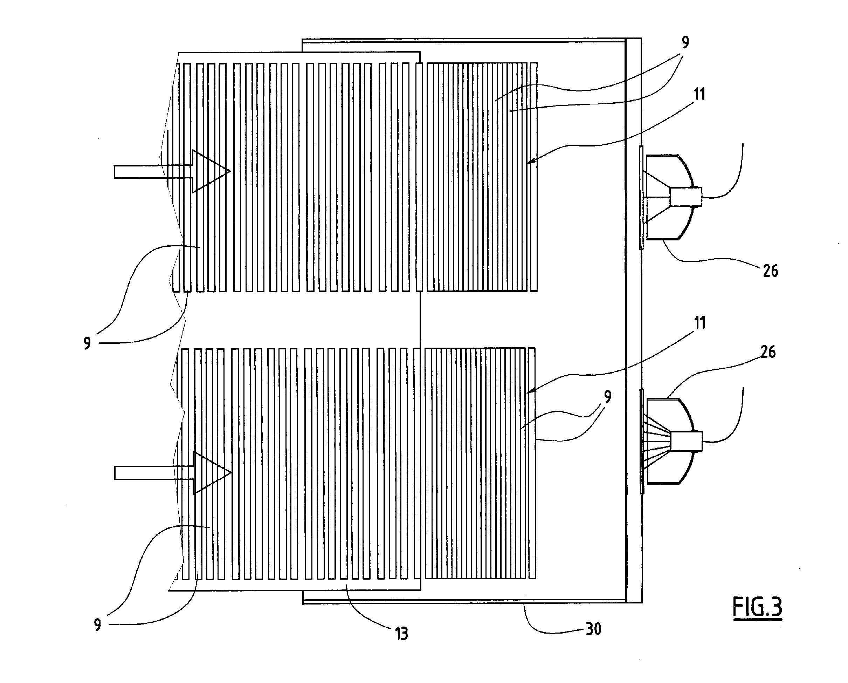

Two (in the illustrated embodiment in figure 3) or more surveillance systems of the invention can be provided, located side-by-side such as commonly to use same means, for example the transfer device for the product 9, located downstream of the inspection space, which is also used by both systems. Also possible is a single surveillance system for surveying more than one conveyor belt advancing the product 9.

Obviously numerous modifications of a practical-applicational nature can be brought to the present invention without its forsaking the inventive idea as claimed herein below.

## Claims

1. A quality production surveillance system of elongate shape comestible pasta product, which comprises:
moving means (12, 13) for causing indeterminate quantities of product to run in a form of a substantially flat and continuous layer within an inspection space (20) wherein the product is inspected;
illuminating means (25, 26) for illuminating the product running in the inspection space (20),
at least a television camera (21) able to acquire a continuous succession of images of the product running in the inspection space (20),
a product transfer device (30) located downstream of the inspection space (20), destined to receive the inspected product,
a product expulsion group (41, 42), acting on a normal flow of the product along the product transfer device (30) to deviate, on command, a limited quantity of the product towards a discharge,
an electronic processing device (51) to control images acquired by the television camera (21) and to command activation of the product expulsion group (41, 42) when it detects at least a single defective product, the commanded activation being such as to expel a limited mass of product containing the single defective product,
**characterised in that** the moving means are able to cause the items of the product, one by one, to roll, with a rotation about the axis thereof, in the inspection space (20).

2. The system of claim 1, **characterised in that** it comprises an inspection surface (11) and the moving means (12, 13) for causing rolling of the product are to advance the product, the said product being arranged with an axis thereof horizontal and transversal to the advancement direction, and to release the product onto the inspection surface (11), such as to realise on the inspection surface a substantially single layer of the product which rolls substantially continuously on the surface (11).

3. The system of claim 2, **characterised in that** the moving means (12, 13) for realising the rolling of the product comprise a slab (12) which is inclined downwards in the advancement direction, an upper surface of which defines the inspection surface (11), and a conveyor device (13), located upstream of the inclined slab (12) and destined to transfer the product onto the slab (12) in a substantially single and continuous layer.

4. The system of claim 1, **characterised in that** the transfer device (30) of the product comprises a descent corridor (31) generally orientated in such a way as to transfer the product in a downwards direction.

5. The system of claim 4, **characterised in that** the expulsion group (41, 42) comprises an obturator (41) which is insertable and de-insertable across the descent corridor, and which is destined to intercept the flow of the product along the corridor.

6. The system of claim 5, **characterised in that** the expulsion group (41, 42) comprises a hatch (42) fashioned in a wall (32, 33) of the corridor, at the obturator (41) position, destined to externally evacuate the product intercepted by the obturator.

7. The system of claim 4, **characterised in that** the longitudinal axis of the descent corridor (31) has a zigzag profile extending in a downwards direction.

8. The system of claim 3, **characterised in that** the slab (12) is translucent and at least an illuminating device (25) is directed against the slab (12), which illuminating device (25) is directed from a lower surface towards an upper surface of the slab (12).

9. The system of claim 1, **characterised in that** it comprises at least an illuminating device (26) destined to provide a substantially diffused light in the inspection space (20), located above the inspection surface (11).

10. The system of claim 9, **characterised in that** the illuminating device (26) exhibits a translucent screen located between the light source and the inspection surface (11).

## Patentansprüche

1. Qualitätsproduktion-Überwachungssystem für ein länglich geformtes essbares Teigwarenprodukt, Folgendes umfassend:
Bewegungsmittel (12, 13), um den Durchlauf unbestimmter Mengen des Produkts in Form einer im Wesentlichen flachen und durchgehenden Schicht in einem Prüfraum (20), in dem das Produkt geprüft wird, zu bewirken,
Beleuchtungsmittel (25, 26) zum Beleuchten des durch den Prüfraum (20) laufenden Produkts,
mindestens eine Fernsehkamera (21), die in der Lage ist, eine fortlaufende Folge von Bildern des durch den Prüfraum (20) laufenden Produkts zu erfassen,
eine Produktüberführungsvorrichtung (30), die prozessabwärts des Prüfraumes (20) angeordnet und dafür vorgesehen ist, das geprüfte Produkt aufzunehmen,
eine Produktausstoßgruppe (41, 42), die auf einen normalen Produktstrom entlang der Produktüberführungsvorrichtung (30) einwirkt, um auf Anweisung eine begrenzte Menge des Produkts hin zu einer Ausgabe zu lenken,
eine elektronische Verarbeitungsvorrichtung (51) zum Steuern von Bildern, die durch die Fernsehkamera (21) erfasst werden, und zum Anweisen der Aktivierung der Produktausstoßgruppe (41, 42), wenn sie mindestens ein einzelnes schadhaftes Produkt erkennt, wobei die angewiesene Aktivierung derart ist, dass eine begrenzte Masse des Produkts, die das einzelne schadhafte Produkt enthält, ausgestoßen wird,
**dadurch gekennzeichnet, dass** die Bewegungsmittel in der Lage sind zu bewirken, dass die Einzelstücke des Produkts mit einer Drehung um ihre Achse nacheinander in den Prüfraum (20) rollen.

2. System nach Anspruch 1, **dadurch gekennzeichnet, dass** es eine Prüffläche (11) umfasst und die Bewegungsmittel (12, 13) für das Bewirken des Rollens des Produkts dazu dienen, das Produkt vorwärts zu bewegen, wobei das Produkt mit seiner Achse horizontal und quer zur Vorwärtsbewegungsrichtung angeordnet ist, und dafür, das Produkt derart auf die Prüffläche (11) auszugeben, dass auf der Prüffläche eine im Wesentlichen einzelne Schicht des Produkts zu erzielen, die im Wesentlichen kontinuierlich auf der Fläche (11) rollt.

3. System nach Anspruch 2, **dadurch gekennzeichnet, dass** die Bewegungsmittel (12, 13) zum Erzielen des Rollens des Produkts eine Platte (12) umfassen, die in Vorwärtsbewegungsrichtung nach unten geneigt ist und deren obere Fläche die Prüffläche (11) definiert, und eine Beförderungsvorrichtung (13), die prozessaufwärts der geneigten Platte (12) angeordnet und dafür vorgesehen ist, das Produkt in einer im Wesentlichen einzelnen und durchgehenden Schicht auf die Platte (12) zu überführen.

4. System nach Anspruch 1, **dadurch gekennzeichnet, dass** die Überführungsvorrichtung (30) für das Produkt einen Fallgang (31) umfasst, der im Allgemeinen derart ausgerichtet ist, dass das Produkt in einer Abwärtsrichtung überführt wird.

5. System nach Anspruch 4, **dadurch gekennzeichnet, dass** die Ausstoßgruppe (41, 42) ein Sperrelement (41) umfasst, das quer in den Fallgang eingeführt und aus diesem entfernt werden kann und das dafür vorgesehen ist, den Produktstrom in dem Gang abzufangen.

6. System nach Anspruch 5, **dadurch gekennzeichnet, dass** die Ausstoßgruppe (41, 42) eine Klappe (42) umfasst, die an der Position des Sperrelements (41) in einer Wand (32, 33) des Ganges gebildet und dafür vorgesehen ist, das durch das Sperrelement abgefangene Produkt nach außen zu verbringen.

7. System nach Anspruch 4, **dadurch gekennzeichnet, dass** die Längsachse des Fallganges (31) ein Zickzackprofil aufweist, dass sich in Abwärtsrichtung erstreckt.

8. System nach Anspruch 3, **dadurch gekennzeichnet, dass** die Platte (12) lichtdurchlässig ist und mindestens eine Beleuchtungsvorrichtung (25) auf die Platte (12) gerichtet ist, wobei die Beleuchtungsvorrichtung (25) von einer unteren Fläche zu einer oberen Fläche der Platte (12) ausgerichtet ist.

9. System nach Anspruch 1, **dadurch gekennzeichnet, dass** es mindestens eine Beleuchtungsvorrichtung (26) umfasst, die dafür vorgesehen ist, ein im Wesentlichen gestreutes Licht im Prüfraum (20) bereitzustellen, und die über der Prüffläche (11) angeordnet ist.

10. System nach Anspruch 9, **dadurch gekennzeichnet, dass** die Beleuchtungsvorrichtung (26) einen lichtdurchlässigen Schirm aufweist, der zwischen der Lichtquelle und der Prüffläche (11) angeordnet ist.

## Revendications

1. Système de surveillance de la qualité de production d'un produit de type pâte comestible à forme allongée, comprenant :
un moyen de déplacement (12, 13) pour provoquer l'écoulement de quantités indéterminées du produit sous forme d'une couche essentiellement plane et continue dans un espace d'inspection (20) dans lequel le produit est inspecté ;
un moyen d'illumination (25, 26) pour éclairer le produit qui s'écoule dans l'espace d'inspection (20),
au moins une caméra de télévision (21) capable d'acquérir une succession d'images continue du produit qui s'écoule dans l'espace d'inspection (20),
un dispositif de transfert de produit (30) agencé en aval de l'espace d'inspection (20), destiné à recevoir le produit inspecté,
un groupe d'expulsion de produit (41, 42) agissant sur un flux normal du produit le long du dispositif de transfert de produit (30) pour dévier, sur commande, une quantité limitée du produit vers une décharge,
un dispositif de traitement électronique (51) pour contrôler des images acquises par la caméra de télévision (21) et pour commander l'activation du groupe d'expulsion de produit (41, 42) lorsqu'il détecte au moins un produit défectueux unique, l'activation commandée étant telle qu'elle expulse une masse limitée de produit contenant le produit défectueux unique,
**caractérisé en ce que** le moyen de déplacement est capable de faire rouler les éléments du produit un par un, avec une rotation autour de leur axe, dans l'espace d'inspection (20).

2. Système selon la revendication 1, **caractérisé en ce qu'**il comprend une surface d'inspection (11), et le moyen de déplacement (12, 13) pour provoquer le roulage du produit est destiné à faire avancer le produit, ledit produit étant agencé avec un de ses axes horizontal et transversal par rapport à la direction d'avancement, et à libérer le produit sur la surface d'inspection (11), de manière à réaliser sur la surface d'inspection une couche de produit essentiellement unique qui roule de manière essentiellement continue sur la surface (11).

3. Système selon la revendication 2, **caractérisé en ce que** le moyen de déplacement (12, 13) pour réaliser le roulage du produit comprend une plaque (12) inclinée vers le bas dans la direction d'avancement dont une surface supérieure définit la surface d'inspection (11), et un dispositif convoyeur (13) agencé en amont de la plaque inclinée (12) et destiné à transférer le produit sur la plaque (12) en une couche essentiellement unique et continue.

4. Système selon la revendication 1, **caractérisé en ce que** le dispositif de transfert (30) du produit comprend un couloir de descente (31) généralement orienté de manière à transférer le produit dans une direction orientée vers le bas.

5. Système selon la revendication 4, **caractérisé en ce que** le groupe d'expulsion (41, 42) comprend un obturateur (41) qui peut être inséré et désinséré en travers du couloir de descente, et qui est destiné à intercepter le flux du produit le long du couloir.

6. Système selon la revendication 5, **caractérisé en ce que** le groupe d'expulsion (41, 42) comprend une trappe (42) formée dans une paroi (32, 33) du couloir, à la position de l'obturateur (41), destinée à évacuer le produit intercepté par l'obturateur vers l'extérieur.

7. Système selon la revendication 4, **caractérisé en ce que** l'axe longitudinal du couloir de descente (31) présente un profil en zigzag qui s'étend dans une direction orientée vers le bas.

8. Système selon la revendication 3, **caractérisé en ce que** la plaque (12) est translucide et **en ce qu'**au moins un dispositif d'illumination (25) est dirigé vers la plaque (12), ledit dispositif d'illumination (25) étant dirigé depuis une surface inférieure de la plaque (12) vers une surface supérieure de celle-ci.

9. Système selon la revendication 1, **caractérisé en ce qu'**il comprend au moins un dispositif d'illumination (26) destiné à procurer une lumière sensiblement diffuse dans l'espace d'inspection (20), agencé au-dessus de la surface d'inspection (11).

10. Système selon la revendication 9, **caractérisé en ce que** le dispositif d'illumination (26) comporte un écran translucide situé entre la source lumineuse et la surface d'inspection (11).
